Europäisches Patentamt

European Patent Office

Office européen des brevets

(11) Publication number: **0 181 304 B1**

# EUROPEAN PATENT SPECIFICATION

(45) Date of publication of patent specification: **30.10.91**   (51) Int. Cl.5: **A61K 7/16**

(21) Application number: **85850350.1**

(22) Date of filing: **30.10.85**

(54) **A rinsing solution intended primarily to prevent the occurrence of oral corrosion in dental alloys.**

(30) Priority: **06.11.84 SE 8405553**

(43) Date of publication of application:
**14.05.86 Bulletin 86/20**

(45) Publication of the grant of the patent:
**30.10.91 Bulletin 91/44**

(84) Designated Contracting States:
**AT BE CH DE FR GB IT LI LU NL SE**

(56) References cited:
**DE-A- 2 260 949**
**US-A- 4 314 990**

**ELECTROCHIMICA ACTA, vol. 25, no. 6, 1980, pages 851-856, Pergamon Press Ltd, GB; H. DO DUC et al.: "Electrochemical behavior of Sn8Hg(gamma-2) and dental amalgam in a phosphate buffer solution"**

(73) Proprietor: **Palaghias, Georgios**
**Spinnrocksvägen 5**
**S-161 48 Bromma(SE)**

(72) Inventor: **Palaghias, Georgios**
**Spinnrocksvägen 5**
**S-161 48 Bromma(SE)**

(74) Representative: **Barnieske, Hans Wolfgang**
**c/o H.W. Barnieske Patentbyrä AB P.O. Box**
**25 Turingegatan 26**
**S-151 21 Södertälje 1(SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 181 304 B1

## Description

The present invention relates to a rinsing solution intended primarily to prevent the occurrence of oral corrosion in dental alloys, wherein said solution contains a phosphate ion donator which forms a protective layer of sparingly soluble metal phosphates with the metal ions precipitated from the dental alloys, along the exposed surfaces, thus preventing further metal precipitation.

A vast number of patients who have received restorative dental treatment complain of a metallic taste, a burning sensation on the tongue, irritation of the mucous membrane, specific or diffuse pains, headaches, etc. Although no epidemiological examination has been carried out, for many of these patients it is likely that the problems are associated with the temporomandibular joint or periodontitis. For other patients the aetiology of the symptoms cannot be related to the condition ascertained in the oral cavity. However, there is still a large group in which the following common features are to be found; debilitated general condition, poor dietary habits, low rate of saliva secretion, low buffer capacity, low pH-value in the saliva, poor oral hygiene and extensive restorative dentistry.

The above symptoms experienced by these patients are said to be caused by corrosion products from dental alloys. It has been ascertained that dental alloys corrode in the oral cavity and that the corrosion products can cause toxic reactions in tissues in their proximity or in other organs in the human body.

Upon closer examination it emerged that certain components do exist in saliva, which exert an inhibiting effect on dental alloys, e.g. phosphate buffer, bicarbonate buffer, polyphosphates and certain organic components. Furthermore, a positive synergism was ascertained if two or more of these components were present in the same solution. The effect has also been found to be augmented by certain ions such as calcium and zinc.

For patients with low saliva secretion levels, poor buffering ability in the saliva or with low pH-value in the oral cavity, however, the inhibiting effect is insufficient.

From ELECTROCHIMICA ACTA, vol. 25 no.6, 1980 pages 851-856 is known that a buffer solution containing phosphate may prevent oral corrosion of a dental alloy.

In order to find out more about the corrosion process of dental alloys, conventional amalgams have been tested in some electrolytes normally occurring in the oral cavity, such as bacteria metabolites, (e.g. acetic acid, formic acid, lactic acid, ammonia), common salt, sodium sulphide, etc. As is kown, these electrolytes are factors which stimulate the corrosion process. In conventional amalgam alloys containing zinc it has been found that, being the most electro-negative element in the alloy, zinc is dissolved out of the amalgam first. When the zinc has been dissolved out the gamma$_2$ phase (Sn-Hg) will be attacked. The tin in the gamma$_2$-phase (Sn-Hg) then forms corrosion products which are difficult to dissolve and will be precipitated on the surface of the amalgam and the quick-silver will react with the gamma phase (Ag$_3$Sn), thus forming new gamma$_1$ (Ag-Hg) and gamma$_2$ (Sn-Hg) phases. Once the zinc has been dissolved out, the rest of the alloy will be attacked.

Long-term studies with non-gamma$_2$ amalgams or amalgams containing a high percentage of copper have also been performed in the above mentioned electrolytes. If such alloys contain zinc, the zinc will be dissolved out first in these amalgams also. In the next stage the copper is attacked and forms soluble corrosion products in most of the electrolytes. There is, for instance, great affinity between copper and ammonia, but also between copper and organic acids.

Surprisingly it has now proved possible by means of the present invention to substantially eliminate the above-mentioned drawbacks of ordinary dental amalgams.

According to the invention, this is achieved by means of the rinsing solution described in the introduction, containing a phosphate ion donator comprising a stabilized supersaturated buffer solution consisting of at least 5-1000 mM/l NaH$_2$PO$_4$ and 5-1000 mM/l Na$_2$HPO$_4$ and 0,5-20 mM/l (NaPO$_3$)$_6$ as a stabilizer.

The rinsing solution should preferably be used when cavities are being filled with amalgam, and for patients with a low level of saliva secretion, poor buffer ability in their saliva or low pH-value in the oral cavity, the rinsing solution should be used regularly even though a certain long-term effect has been noted.

The invention can be used for all dental alloys known hitherto.

Although the inhibiting effect on the corrosion process of the dental amalgam achieved according to the invention has not yet fully expounded, it is assumed that the phosphate ion donator exerts an inhibiting effect by, in contact with primarily zinc, forming a protective layer of sparingly soluble zinc phosphates on the exposed surfaces of the amalgam filling, which in turn protects the amalgam filling from further metal precipitation. Similarly, the phosphate ion donator according to the invention forms sparingly soluble copper phosphates when in contact with copper.

The rinsing solution is supersaturated and acidferous. The solution is provided to the surface of the

2

dental alloy to activate said surface and to precipitate the protective layer under the elevated temperature. The rinsing solution contains a buffer solution which comprises 5 - 1000 mM/l $NaH_2PO_4$ together with 5 - 1000 mM/l $Na_2HPO_4$. To activate a stabilized solution the buffer solution can be comprised of

| | | |
|---|---|---|
| 5 - 1000 | mM/1 | $NaH_2PO_4$ |
| 5 - 1000 | mM/1 | $Na_2HPO_4$ |
| 0,5 - 20 | mM/1 | $(NaPO_3)_6$ |
| 5 - 1000 | mM/1 | $NaHCO_3$ |
| 0,5 - 5 | mM/1 | $CaCl_2$ |
| 0,5 - 5 | mM/1 | $ZnCl_2$ |

A preferred durable solution may be comprised of

| | | |
|---|---|---|
| 30 | mM/1 | $NaH_2PO_4$ |
| 30 | mM/1 | $Na_2HPO_4$ |
| 2,5 | mM/1 | $(NaPO_3)_6$ |
| 15 | mM/1 | $NaHCO_3$ |
| 1,5 | mM/1 | $CaCl_2$ |
| 0,15 | mM/1 | $ZnCl_2$ |

The inhibiting effect established according to the invention is thus achieved by the phosphate ion donator forming complex phosphate compounds as follows:

Zinc and copper are easily dissolved out of the amalgam and are only attacked in corrosive electrolytes. Particularly in the presence of water, oxygen and phosphate solutions, permanent phosphate films are formed consisting of $Zn_3(PO_3)_2$ and $Cu_3(PO_4)_2$.

In the case of tin and tin alloys the inhibiting effect has been found to be achieved by a layer of phosphate on the surface consisting of tin, oxygen and phosphate atoms which are strongly bound with covalent bonds. These phosphate layers prevent the release of tin ions and are the cause of an improvement in the alloy. This improvement is expressed in a positive shift of the potential.

The proposed addition of $(NaPO_3)_6$ exerts an inhibiting effect on the corrosion process and on the precipitation of carbonates in the pH-range normally prevailing in the oral cavity. Thus, in this pH-range the polyphosphate is hydrolyzed and precipitates out in the form of orthophosphate. Furthermore, the addition of $CaCl_2$ and $ZnCl_2$ increases the adhesion properties of the solutions and its ability to form sparingly soluble compounds on the metal surfaces. At a pH-value of about 5,5 and lower the precipitation of quicksilver is prevented by mercury carbonates being formed with the proposed additive $NaHCO_3$.

The invention will be described more fully in the following with reference to the example below.

Example 1

Three amalgam alloys of different types and systems were selected, having the following compositions: (the percentages are by weight)

3

|  | I | II | III |
|---|---|---|---|
| Silver | 68,5% | 70% | 45,8% |
| Tin | 26% | 16% | 29% |
| Zinc | 0,5% | 1% | 0,2% |
| Copper | 5% | 13% | 25% |
| Quick-silver/ alloy ratio | 1/1 | 1/1 | 1/1 |

Four test bodies of each alloy were manufactured in known manner by mixing the metal powder with quick-silver. These test bodies were immersed in glass jars containing 50 ml of an electrolyte consisting of

| | | |
|---|---|---|
| 15 | mM/l | $NaH_2PO_4$ |
| 15 | mM/l | $Na_2HPO_4$ |
| 2,5 | mM/l | $(NaPO_3)_6$ |
| 15 | mM/l | $NaHCO_3$ |
| 1,5 | mM/l | $CaCl_2$ |
| 0,15 | mM/l | $ZnCl_2$ |

The electrolyte was exchanged once a month over a three-month period and was analyzed for the occurrence of silver, copper, zinc, quick-silver and tin. The results are given in the following tables. These experiments were repeated with the same electrolyte to which 0,5% by weight NaCl had been added. The results obtained did not differ from those given below. Satisfactory experiments have also been performed using up to 1000 mM/l of $NaH_2PO_4$ and of $Na_2HPO_4$.

I

| Month | Cu | Hg | Zn | Ag | Sn |
|---|---|---|---|---|---|
| 1 | <0,03 | <0,01 | 9,09 | <0,03 | <0,8 |
| 2 | <0,03 | <0,01 | 9,00 | <0,03 | <0,8 |
| 3 | <0,03 | <0,01 | 7,28 | <0,03 | <0,8 |

(the precipitation is stated in mg/l)

II

| Month | Cu | Hg | Zn | Ag | Sn |
|---|---|---|---|---|---|
| 1 | 0,06 | <0,01 | 9,52 | <0,03 | <0,8 |
| 2 | <0,03 | 0,05 | 8,39 | <0,03 | <0,8 |
| 3 | 0,05 | <0,01 | 7,50 | <0,03 | <0,8 |

(the precipitation is stated in mg/l)

III

| Month | Cu | Hg | Zn | Ag | Sn |
|---|---|---|---|---|---|
| 1 | 0,06 | <0,01 | 9,05 | <0,03 | <0,8 |
| 2 | <0,03 | 0,03 | 8,39 | <0,03 | <0,8 |
| 3 | <0,03 | <0,01 | 7,46 | <0,03 | <0,8 |

(the precipitation is stated in mg/l)

**Claims**

1. A rinsing solution intended primarily to prevent the occurrence of oral corrosion in dental alloys, wherein said solution contains a phosphate ion donator which forms a protective layer of sparingly soluble metal phosphates with the metal ions precipitated from the dental alloys, along the exposed surfaces, thus preventing further metal precipitation, **characterized** in that said phosphate ion donator comprises a stabilized supersaturated buffer solution consisting of at least 5 - 1000 mM/l $NaH_2PO_4$ and 5 - 1000 mM/l $Na_2HPO_4$ and 0,5 - 20 mM/l $(NaPO_3)_6$ as a stabilizer.

2. A rinsing solution according to claim 1, **wherein** the buffer solution also includes $NaHCO_3$, $CaCl_2$ and $ZnCl_2$.

3. A rinsing solution according to claim 1 - 2, **wherein** the components included in the buffer solution comprise:

| | | |
|---|---|---|
| 5 - 1000 | mM/l | $NaH_2PO_4$ |
| 5 - 1000 | mM/l | $Na_2HPO_4$ |
| 0,5 - 20 | mM/l | $(NaPO_3)_6$ |
| 5 - 1000 | mM/l | $NaHCO_3$ |
| 0,5 - 5 | mM/l | $CaCl_2$ |
| 0,5 - 5 | mM/l | $ZnCl_2$. |

4. A rinsing solution according to claim 3, **wherein** the components included in the buffer solution comprise:

| | | |
|---|---|---|
| 30 | mM/l | $NaH_2PO_4$ |
| 30 | mM/l | $Na_2HPO_4$ |

| 2,5 | mM/l | $(NaPO_3)_6$ |
|---|---|---|
| 15 | mM/l | $NaHCO_3$ |
| 1,5 | mM/l | $CaCl_2$ |
| 0,15 | mM/l | $ZnCl_2$ |

**Revendications**

1. Solution rinçante destinée à la prévention de la corrosion orale dans des alliages dentaires, ladite solution renfermant un donneur d'ions de phosphate, ledit donneur d'ions de phosphate et les ions métallique précipités des alliages dentaires formant une couche de protection des phosphates métalliques difficilement solubles sur la surface d'alliage dentaire, ainsi supprimant une précipitation ultérieure, **charactérisée en ce** que ledit donneur d'ions de phosphate renferme une solution tampon sursaturée et stabilisée, ladite solution tampon renfermant le minimum 5 - 1000 mM/l $NaH_2PO_4$ et 5 - 1000 mM/l $Na_2HPO_4$ et 0,5 - 20 mM/l $(NaPO_3)_6$ comme stabilisateur.

2. Solution rinçante selon la revendication 1, characterisée en ce que ladite solution tampon renferme $NaHCO_3$, $CACl_2$ et $ZnCl_2$.

3. Solution rinçante selon la revendication 1 ou 2, characterisée en ce que les constituants renfermés dans la solution tampon renferment

| 5 - 1000 mM/l | $NaH_2PO_4$ |
|---|---|
| 5 - 1000 mM/l | $Na_2HPO_4$ |
| 0,5 - 20 mM/l | $(NaPO_3)_6$ |
| 5 - 1000 mM/l | $NaHCO_3$ |
| 0,5 - 5 mM/l | $CaCl_2$ |
| 0,5 - 5 mM/l | $ZnCl_2$ |

4. Solution rinçante selon la revendication 1, charactérisée en ce que les constituants renfermés dans la solution tampon renferment

| 30 | mM/l | $NaH_2PO_4$ |
|---|---|---|
| 30 | mM/l | $Na_2HPO_4$ |
| 2,5 | mM/l | $(NaPO_3)_6$ |
| 15 | mM/l | $NaHCO_3$ |
| 1,5 | mM/l | $CaCl_2$ |
| 0,15 | mM/l | $ZnCl_2$ |

**Patentansprüche**

1. Spüllösung zur Verhinderung der Korrosion von Dentallegierungen im Mund mit einem Phosphationendonator, wobei der Phosphationendonator mit von der Dentallegierung ausgeschiedenen Metallionen eine Schutzschicht aus schwerlöslichen Metallphosphaten bildet und so eine weitere Ausscheidung von Metallionen an der Oberfläche der Dentallegierung verhindert, **dadurch gekennzeichnet,** daß der Phosphationenendonator eine stabilisierte übersättigte Pufferlösung aufweist und die Pufferlösung mindestens 5 - 1000 mM/l $NaH_2PO_4$ und 5 - 1000 mM/l $Na_2HPO_4$ und 0,5 - 20 mM/l $(NaPO_3)_6$ als Stabilisator enthält.

2. Spüllösung nach Anspruch 1, dadurch gekennzeichnet, daß die Pufferlösung auch $NaHCO_3$, $CaCl_2$ und $ZnCl_2$ enthält.

3. Spüllösung nach Anspruch 1 oder 2, dadurch gekennzeichnet, daß die Komponenten der Pufferlösung

| | |
|---|---|
| 5 - 1000 mM/l | $NaH_2PO_4$ |
| 5 - 1000 mM/l | $Na_2HPO_4$ |
| 0,5 - 20 mM/l | $(NaPO_3)_6$ |
| 5 - 1000 mM/l | $NaHCO_3$ |
| 0,5 - 5 mM/l | $CaCl_2$ |
| 0,5 - 5 mM/l | $ZnCl_2$ |

enthalten.

4. Spüllösung nach Anspruch 3, dadurch gekennzeichnet, daß die Komponenten der Pufferlösung

| | | |
|---|---|---|
| 30 | mM/l | $NaH_2PO_4$ |
| 30 | mM/l | $Na_2HPO_4$ |
| 2,5 | mM/l | $(NaPO_3)_6$ |
| 15 | mM/l | $NaHCO_3$ |
| 1,5 | mM/l | $CaCl_2$ |
| 0,15 | mM/l | $ZnCl_2$ |

enthalten.

7